# EUROPEAN PATENT SPECIFICATION

(11) **EP 1 200 661 B1**
(45) Date of publication and mention of the grant of the patent: **21.07.2004**
(21) Application number: 00952338.2
(22) Date of filing: 01.08.2000
(51) Int. Cl.: D04H 13/00, D01D 5/098, D01D 5/30, D04H 1/42, D04H 1/56

(54) **COMPOSITE NONWOVEN SHEET MATERIAL**
VERBUNDVLIESMATERIAL
MATERIAU COMPOSITE NON TISSE EN FEUILLE

(30) Priority: 02.08.1999 US 146896 P
(43) Date of publication of application: 02.05.2002
(73) Proprietor: E.I. DU PONT DE NEMOURS AND COMPANY, Wilmington, Delaware 19898 (US)
(72) Inventor: RUDISILL, Edgar, N., Nashville, TN 37204 (US); BANSAL, Vishal, Richmond, VA 23233 (US); DAVIS, Michael, C., Midlothian, VA 23113 (US)
(74) Representative: Beacham, Annabel Rose
(86) International application number: PCT/US2000/020882
(87) International publication number: WO 2001/009425

(56) References cited:
- WO-A-00/37723
- US-A- 5 415 925
- US-A- 5 484 645
- US-A- 5 503 907
- US-A- 5 554 437
- US-A- 5 616 408
- US-A- 5 885 909

## Description

### BACKGROUND OF THE INVENTION

### Field of the Invention

This invention relates to nonwoven fabrics, and more specifically, to composite nonwoven fabrics that include a barrier layer of fine meltblown fibers. The nonwoven fabrics of the invention are suited for use in apparel, wipes, hygiene products, and medical products such as surgical gowns and drapes, bandages. sterilization wraps, and wound dressings.

### Description of Related Art

Thermoplastic resins have been extruded to form fibers for many years. These resins include polyolefins, polyesters, polyamides, and polyurethanes. The extruded fibers have been made into a variety of nonwoven fabrics including composite laminates such as spunbond-meltblown-spunbond ("SMS") composite sheets. In SMS composites, the exterior layers are spunbond fiber layers that contribute strength to the overall composite, while the core layer is a meltblown fiber layer that provides barrier properties. Traditionally, the spunbond and meltblown layers of SMS composites have been made of polypropylene fibers.

For certain end use applications, such as medical gowns, it is desirable that SMS composite sheets have good strength and barrier properties, while also being as soft and drapeable as possible. While polypropylene-based SMS fabrics offer good strength and barrier properties, they tend not to be as soft and drapeable as is desirable for apparel products. Polypropylene-based SMS fabrics also have the limitation that they cannot be sterilized with gamma radiation because such fabrics are discolored and weakened when sterilized with gamma radiation, and because gamma radiation sterilization of polypropylene-based SMS fabrics generates unpleasant odors. A polymer fiber or fabric is generally considered to be not radiation sterilizable when sterilization of the fabric with gamma radiation causes a significant reduction in the strength of the fiber or fabric, noticeably changes the appearance of the fiber or fabric, or generates an objectionable odor. This inability to undergo gamma radiation sterilization presents a significant problem for polypropylene-based SMS fabrics because radiation sterilization is commonly used throughout the medical industry.

U.S. Patent Nos. 5,484,645 and 5,498,463 disclose SMS composite fabrics having a polyethylene meltblown layer sandwiched between spunbond layers. In U.S. Patent No. 5,484,645, the spunbond layers are comprised of bicomponent fibers meltspun from polyethylene and a higher melting temperature polymer such as polyester. In U.S. Patent No. 5,498,463, the spunbond layers are comprised of polyethylene, polypropylene or bicomponent polyethylene/polypropylene meltspun fibers. It has proven difficult to consistently produce SMS fabrics with desirable barrier properties when the meltblown fiber core is made of polyethylene fibers. This is probably because polyethylene fibers are not as fine as required to make a web with the barrier properties needed for many end use applications.

U.S. Patent No. 5,616,408 discloses an SMS composite fabnc in which the meltblown fibers are compnsed of a blend of polyethylene and a polyethylene processing stabilizing component. The stabilizing component is added to the polyethylene so as to stiffen the soft, highly elongatable polyethylene resin so that the resin can be meltblown without substantial formation of shot, polymer globules and the like. The stabilizing component is disclosed as being another polymer such as a polyolefin, polyester or polyamide added to the polyethylene in an amount of about 1 to 15 percent by weight based upon the weight to the polyethylene polymer. Alternatively, the stabilizing component is disclosed as being a polyethylene crosslinking agent added to the polyethylene in an amount between 0.05 and 1 percent by weight based on the weight of the polyethylene polymer. While meltblowing fibers from a blend of polyethylene and a stabilizing component such a polyester has been found to result in the generation of less fly and shot, meltblown layers compnsed of such polyethylene blends exhibit lower bamer properties than is desirable for the meltblown layer of SMS fabrics designed for end use applications where barrier properties are important.

### BRIEF SUMMARY OF THE INVENTION

A composite sheet is provided that includes a first fibrous layer having a first side and an opposite second side, and a second fibrous layer bonded to the first side of the first fibrous layer. The first fibrous layer is a multiple component meltblown web comprised of at least 95% by weight of meltblown fibers having an average effective diameter of less than 10 microns. The multiple component meltblown web is comprised of 10% to 98% by weight of a first polymer component and 90% to 2% by weight of a second polymer component distinct from said first polymer component. The first polymer component is at least 85% by weight polyethylene and the second polymer component is a polyester polymer. The second fibrous layer is compnsed of at least 95% by weight of second layer fibers having an average effective diameter that is greater than the average effective diameter of the meltblown fibers of the first fibrous layer. The composite sheet has a basis weight of less than 120 g/m², a machine direction and a cross direction grab tensile strength of at least 35 N, and a hydrostatic head of at least 42,5 cm.

According to the invention, the second polymer component of the multiple component meltblown web is polyester polymer, preferably selected from the group of poly(ethylene terephthalate), poly(trimethylene terephthalate), poly(butylene terephthalate), and copolymers and terpolymers thereof.

According to the invention, at least 10% of the fibers in the multiple component meltblown web are bicomponent fibers having a length, and the first and second polymer components are arranged in such bicomponent fibers in a manner such that the first and second polymer components each extend substantially the complete length of the bicomponent fibers. Preferably, the first and second polymer components of the bicomponent fibers are arranged in a side-by-side arrangement wherein the first and second polymer components each extend substantially the complete length of the first layer fibers.

According to the preferred embodiment of the invention, the second layer fibers are substantially continuous meltspun fibers having an average effective diameter of at least 5 microns. Preferably, the second layer fibers are multiple component meltspun fibers that include a polyethylene polymer and another distinct second polymer component, wherein the polyethylene polymer component comprises at least 5% by weight of said second layer fibers. The second polymer component of the second layer fibers is preferably selected from the group of polyester, polyamide, polyurethane, polypropylene, and polystyrene polymers. The meltspun fibers of the second layer fibers preferably have an average effective diameter in the range of 6 to 10 microns, a machine direction and cross direction grab tensile strength of at least 35 Newtons, and a hydrostatic head of at least 3 cm. The meltspun fibers of the second layer fibers may be sheath core bicomponent fibers having a polyethylene sheath and a polyester core. The composite sheet of the invention preferably includes a third fibrous layer bonded to the second side of said first fibrous layer. The third fibrous layer is preferably comprised of substantially continuous meltspun fibers having an average effective diameter of at least 5 microns. Preferably, the first fibrous layer is thermally bonded to said second and third fibrous layers.

The present invention is also directed to garment made from the composite sheet material described above and to a process for making the composite sheet material described above.

### BRIEF DESCRIPTION OF THE DRAWINGS

The accompanying drawings, which are incorporated in and constitute a part of this specification, illustrate the presently preferred embodiments of the invention.
Figure 1 is a diagrammatical cross-sectional view of a composite nonwoven fabric in accordance with one embodiment of the invention.
Figure 2 is a diagrammatical cross-sectional view of a composite nonwoven fabric in accordance with another embodiment of the invention.
Figure 3 is a schematic diagram of a portion of an apparatus used for producing meltblown fibers for use in the composite nonwoven fabric of the invention.
Figure 4 is a schematic illustration of an apparatus for producing a spunbond nonwoven layer for use in the composite nonwoven fabric of the invention.
Figure 5 is schematic illustration of an apparatus for producing the composite nonwoven fabric of the invention.
Figure 6 is a graph of the hydrostatic barrier properties of a number of nonwoven composite sheets made with a meltblown bicomponent web having various combinations of polyester and polyethylene components.
Figure 7 is a schematic diagram of a portion of an apparatus used for producing meltblown fibers for use m the composite nonwoven fabric of the invention.

### DEFINITIONS

The term "polymer" as used herein, generally includes but is not limited to, homopolymers, copolymers (such as for example, block, graft, random and alternating copolymers), terpolymers, etc. and blends and modifications thereof. Furthermore, unless otherwise specifically limited, the term "polymer" shall include all possible geometrical configurations of the material. These configurations include, but are not limited to isotactic, syndiotactic and random symmetries.

The term "polyolefin" as used herein, is intended to mean any of a series of largely saturated open chain polymeric hydrocarbons composed only of carbon and hydrogen. Typical polyolefins include, but are not limited to, polyethylene, polypropylene, polymethylpentene and various combinations of the ethylene, propylene, and methylpentene monomers.

The term "polyethylene" as used herein is intended to encompass not only homopolymers of ethylene, but also copolymers wherein at least 85% of the recurring units are ethylene units.

The term "polypropylene" as used herein is intended to embrace not only homopolymers of propylene but also copolymers where at least 85% of the recurring units are propylene units.

The term "polyester" as used herein is intended to embrace polymers wherein at least 85% of the recurring units are condensation products of carboxylic acids and dihydroxy alcohols with polymer linkages created by formation of an ester unit. This includes, but is not limited to, aromatic, aliphatic, saturated, and unsaturated acids and di-alcohols. The term "polyester" as used herein also includes copolymers (such as block, graft, random and alternating copolymers), blends, and modifications thereof. A common example of a polyester is poly(ethylene terephthalate) which is a condensation product of ethylene glycol and terephthalic acid.

The term "meltblown fibers" as used herein, means fibers formed by extruding a molten thermoplastic polymer through a plurality of fine, usually circular, capillaries as molten threads or filaments into a high velocity gas (e.g. air) stream. The high velocity gas stream attenuates the filaments of molten thermoplastic polymer material to reduce their diameter to between 0.5 and 10 microns. Meltblown fibers are generally discontinuous fibers. Meltblown fibers carried by the high velocity gas stream are normally deposited on a collecting surface to form a web of randomly dispersed fibers.

The term "meltspun fibers" as used herein means small diameter fibers which are formed by extruding molten thermoplastic polymer material as filaments from a plurality of fine. usually circular, capillaries of a spinnerette with the diameter of the extruded filaments then being rapidly reduced. Meltspun fibers are generally continuous and have an average diameter of greater than about 5 microns.

The term "nonwoven fabric, sheet or web" as used herein means a structure of individual fibers or threads that are positioned in a random manner to form a planar material without an identifiable pattern, as in a knitted fabric.

The term "multiple component meltblown web" as used herein means meltblown fibers spun from fine capillaries as molten filaments containing multiple and distinct polymer components, which molten filaments are attenuated by a high velocity gas stream and deposited on a collecting surface as a web of randomly dispersed fibers.

As used herein, the "machine direction" is the long direction within the plane of a sheet, i.e., the direction in which the sheet is produced. The "cross direction" is the direction within the plane of the sheet that is perpendicular to the machine direction.

### TEST METHODS

In the description above and in the non-limiting examples that follow, the following test methods were employed to determine various reported characteristics and properties. ASTM refers to the American Society for Testing and Materials, and AATCC refers to the American Association of Textile Chemists and Colorists.

Basis Weight is a measure of the mass per unit area of a fabric or sheet and was determined by ASTM D-3776, which is hereby incorporated by reference, and is reported in g/m².

Grab Tensile Strength is a measure of the breaking strength of a sheet and was conducted according to ASTM D 5034, and is reported in Newtons.

Elongation of a sheet is a measure of the amount a sheet stretches prior to failure (breaking) in the grab tensile strength test and was conducted according to ASTM D 5034, and is reported as a percent.

Hydrostatic Head is a measure of the resistance of the sheet to penetration by liquid water under a static pressure. The test was conducted according to AATCC-127, and is reported in centimeters.

Frazier Air Permeability is a measure of air flow passing through a sheet under at a stated pressure differential between the surfaces of the sheet and was conducted according to ASTM D 737, and is reported in m³/min/m².

Water Impact is a measure of the resistance of a sheet to the penetration of water by impact and was conducted according to AATCC 42-1989. which is hereby incorporated by reference, and is reported in grams.

### DETAILED DESCRIPTION OF THE INVENTION

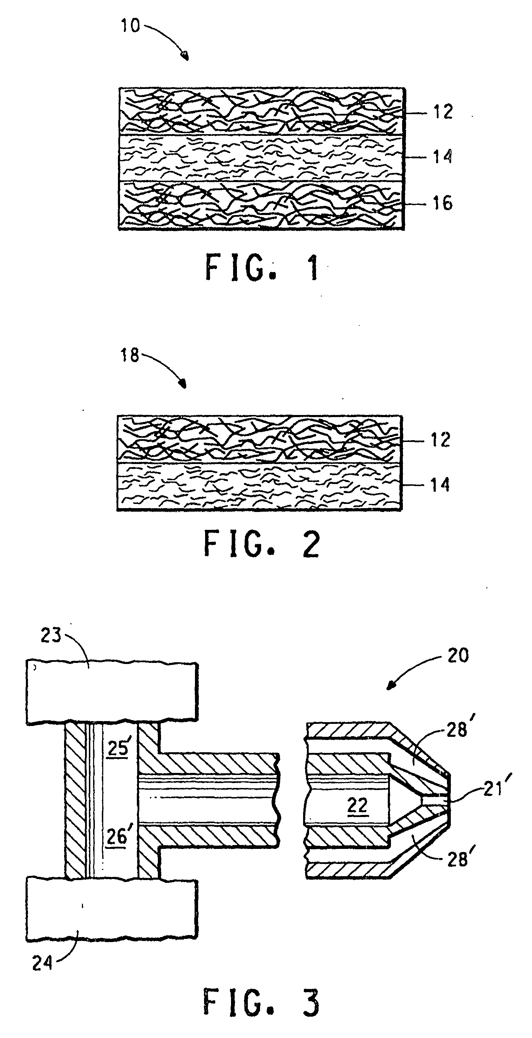

Reference will now be made in detail to the presently preferred embodiments of the invention, examples of which are illustrated below. A composite nonwoven sheet of the invention is shown in Figure 1. The sheet 10 is a three layer composite fabric in which an inner layer 14 comprised of very fine polymer fibers is sandwiched between outer layers 12 and 16, which are each comprised of larger and stronger and bonded fibers. The very fine fibers of inner layer 14, when formed into the layer 14, produce a barrier layer with extremely fine passages. The layer 14 acts as a barrier to fluids but does not prevent the passage of moisture vapor. The bonded fiber layers 12 and 16 are comprised of coarser and stronger fibers that contribute strength, and in some instances barrier, to the composite sheet. The composite sheet of the invention may alternatively be formed as a two layer composite 18, as shown in Figure 2. In the two layer composite sheet, the fine fiber layer 14 is attached on just one side to the coarser and stronger bonded layer 12. According to other alternative embodiments of the invention, the composite sheet may be made with multiple layers of fine fibers like the layer 14. or it may be made with more than two layers of coarser and stronger fiber layers like the layers 12 and 16.

The fine fiber layer 14 comprises a multiple component meltblown web. The multiple component meltblown web is formed from at least two polymers simultaneously spun from a senes of spinning orifices. Preferably, the multiple component meltblown web is a bicomponent web made from two polymers. The configuration of the fibers in the bicomponent web is preferably a side-by-side arrangement in which most of the fibers are made of two side-by-side polymer components that extend for substantially the whole length of each fiber. Alternatively, these bicomponent fibers may have a sheath/core arrangement wherein one polymer is surrounded by another polymer, an "islands-in-the-sea" arrangement in which multiple strands of one polymer are imbedded in another polymer, or any other multiple component fiber structure. Without wishing to be bound by theory, it is believed that the attenuation of the meltblown fibers may actually fracture the multiple component filaments into even finer filaments, some of which may contain only one polymer component. The fibers in the multiple component meltblown web of the layer 14 are typically discontinuous fibers having an average effective diameter of between about 0.5 microns and 10 microns, and more preferably between about 1 and 6 microns, and most preferably between about 2 and 4 microns. As used herein, the "effective diameter" of a fiber with an irregular cross section is equal to the diameter of a hypothetical round fiber having the same cross sectional area.

According to one preferred embodiment of the invention, the first polymer component of the multiple component meltblown web of the layer 14 is comprised of at least 85% polyethylene. Preferably the polyethylene is a linear low density polyethylene having a melt index of at least 10 g/ 10 min (measured according to ASTM D-1238; 2.16 kg @ 190° C), an upper limit melting range of about 120° to 140°C, and a density in the range of 0.86 to 0.97 gram per cubic centimeter. A particularly preferred polyethylene is a linear low density polyethylene with a melt index of 150 g/10 minutes (according to ASTM D-1238) available from Dow Chemical as ASPUN 6831 A. According to another embodiment of the invention, the first polymer component may be an ethylene copolymer such as ethylene vinyl acetate ("EVA"), ethylene methylacrylate ("E-MA"), or SURLYN® ethylene copolymer (available from DuPont, Wilmington, DE). The polyethylene component of the meltblown web of the layer 14 preferably comprises between about 20% and 98% by weight of the web in the fine fiber layer 14, and more preferably comprises between about 55% and 98% by weight of the web in the fine fiber layer 14, and most preferably comprises between about 65% and 97% by weight of the web in the fine fiber layer 14. Alternatively, the polyethylene component of the meltblown web of the layer 14 preferably may comprises as little as between about 10% and 20% by weight of the web in the fine fiber layer 14

According to the invention, the second polymer component of the multiple component meltblown web of layer 14 compnses one or more fiber forming synthetic polymers having a melt temperature greater than 140° C or a glass transition temperature greater than 40° C. Preferably, the other polymer or polymers are gamma radiation stable polymers that improve the spinning of the polyethylene fiber component, such as a polyester, polyamide, polyurethane, or polystyrene polymer. Alternatively, the second polymer component of the fine fiber layer 14 can be a non-radiation sterilizable polymer such as polypropylene if the end use for the sheet does not require that the sheet be radiation sterilizable. The most preferred polymer for the second polymer component of the multiple component meltblown web of layer 14 is a polyester polymer such as poly(ethylene terephthalate), poly(trimethylene terephthalate), poly(butylene terephthalate), and copolymers and terpolymers thereof. A polyester that has been advantageously utilized in the second polymer component of the meltblown web of layer 14 has been poly(ethylene terephthalate) having an intrinsic viscosity of 0.53 (as measured in U.S. Patent 4,743,504) and available from DuPont as Crystar® polyester (Merge 3949).

According to the preferred embodiment of the invention, the fine fibers of the layer 14 are meltblown fibers that are produced according to a meltblowing process. In the meltblowing process, one or more extruders supply melted polymer to a die tip where the polymer is fiberized as it passes through fine capillary openings to form a curtain of filaments. The filaments are pneumatically drawn and normally broken by a jet of air around the fine capillary openings in the die. The fibers are deposited on a moving belt or screen, a scrim, or another fibrous layer. Fibers produced by melt blowing are generally discontinuous fibers having an effective diameter in the range of about 0.5 to about 10 microns.

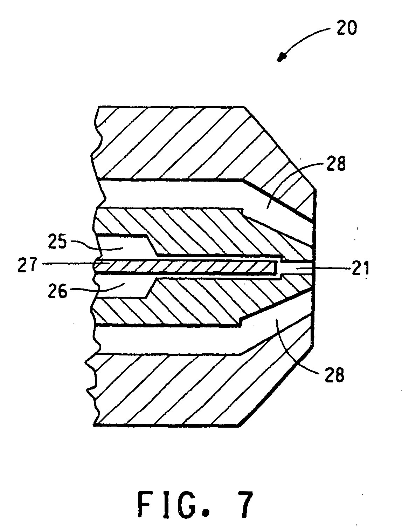

The fibers of the multiple component meltblown web of the layer 14 can be meltblown using a meltblowing apparatus having capillary die openings like that shown in Figure 7. In the sectional view of a meltblowing spin block 20 shown in Figure 7, two different polymeric components are melted in parallel extruders (not shown) and metered separately by gear pumps (not shown) to conduits 25 and 26 that are divided from each other by a plate 27. The polymer components are then fed to a line of capillary orifices 21. Alternatively, the fibers of the multiple component meltblown web of the layer 14 can be meltblown using a meltblowing apparatus having capillary die openings like that shown in Figure 3 and more fully described in U.S. Patent No. 4,795,668. In the sectional view of a meltblowing die 20' shown in Figure 3, two different polymeric components are melted in parallel extruders 23 and 24 and metered separately through gear pumps (not shown) and conduits 25' and 26' into the die cavity 22. From the die cavity, the polymer components are extruded together through a line of capillary orifices 21'. According to another alternative, the polymer components can be fed, in an already layered form, into the cavity of the a spin block from which the capillary orifices are supplied with a multiple component polymer stream.

After exiting the capillary orifices, a jet of hot air supplied from the channels 28 (Figure 7) or the channels 28' (Figure 3) attenuates the emerging polymer filaments. Without wishing to be bound by theory, it is believed that the air jet may fracture the filaments into even finer filaments. The resulting filaments are believed to include bicomponent filaments in which each filament is made of two separate polymer components that both extend the length of the meltblown fiber in a side-by-side configuration. It is aiso believed that some of the fractured filaments may contain just one polymer component. The fine fibers of layer 14 could alternatively be produced by other know meltblowing processes, as for example by the process wherein an individual air nozzle surrounds each polymer capillary, as disclosed in U.S. Patent No. 4,380,570.

The preferred multiple component meltblown web of layer 14 is a bicomponent meltblown web comprised of polyethylene and polyester. The polyethylene component may comprise from 10% to 98% by weight of the meltblown web. Preferably, the polyethylene component comprises from 20% to 98% by weight of the meltblown web and the polyester component comprises from 2% to 80% by weight of the meltblown web. More preferably, the polyethylene component comprises from 55% to 98% by weight of the meltblown web and the polyester component comprises from 2% to 45% by weight of the meltblown web. Even more preferably, the polyethylene component comprises from 65% to 97% by weight of the meltblown web and the polyester component comprises from 3% to 35% by weight of the meltblown web. Most preferably, the polyethylene component comprises from 80% to 95% by weight of the meltblown web and the polyester component comprises from 5% to 20% by weight of the meltblown web.

According to the invention, the larger and stronger bonded fibers of the layers 12 and 16 are conventional meltspun fibers or some other type of strong spunbond fiber. Preferably, the meltspun fibers are substantially continuous fibers. Alternatively, the layers 12 and 16 could be an air-laid or wet-laid staple fiber web or a carded web wherein the fibers are bonded to each other to form a strong web structure. The fibers of layers 12 and 16 should be made of a polymer to which polyethylene-containing fine fibers of the core layer 14 can readily bond. The fibers of layers 12 and 16 are preferably gamma radiation sterilizable in that they have an outer layer comprised of a polymer other than polypropylene, such as polyester, polyethylene, polyamide, or some combination thereof. Where the composite fabric will not be used in end use applications where radiation sterilization is used, the fibers of layers 12 and 16 could also be comprised of a polymer such as polypropylene that is not gamma radiation sterilizable.

A preferred meltspun fiber for the layers 12 and 16 is a bicomponent fiber comprised of polyester and polyethylene. The polyester component contributes to the strength to the fabric while the polyethylene component makes the fabric softer and more drapable. In addition, the polyethylene component has a lower melting temperature than the polyester component of the fiber so as to make the fiber layers 12 and 16 more readily bondable to the fine fibers of the core layer 14 using a thermal bonding process. Alternatively, layers 12 and 16 could be comprised of a blend of single polymer component fibers, as for example, a spunbond web wherein a portion of the fibers are polyethylene fibers and a portion of the fibers are polyester fibers.

According to the preferred embodiment of the invention, the larger and stronger fibers of the layers 12 and 16 are substantially continuous spunbonded fibers produced using a high speed melt spinning process, such as the high speed spinning processes disclosed in U.S. Patent Nos. 3,802,817; 5,545,371; and 5,885,909. According to the preferred high speed melt spinning process, one or more extruders supply melted polymer to a spin block where the polymer is fibenzed as it passes through openings to form a curtain of filaments. The filaments are partially cooled in an air quenching zone. The filaments are then pneumatically drawn to reduce their size and impart increased strength. The filaments are deposited on a moving belt, scnm or other fibrous layer. Fibers produced by the preferred high speed melt spinning process are substantially continuous and have a diameter of from 5 to 30 microns. These fibers can be produced as single component fibers, as multiple component fibers, or as some combination thereof. Multicomponent fibers can be made in vanous known cross-sectional configurations, including side-by-side, sheath-core, segmented pie, or islands-in-the-sea configurations.

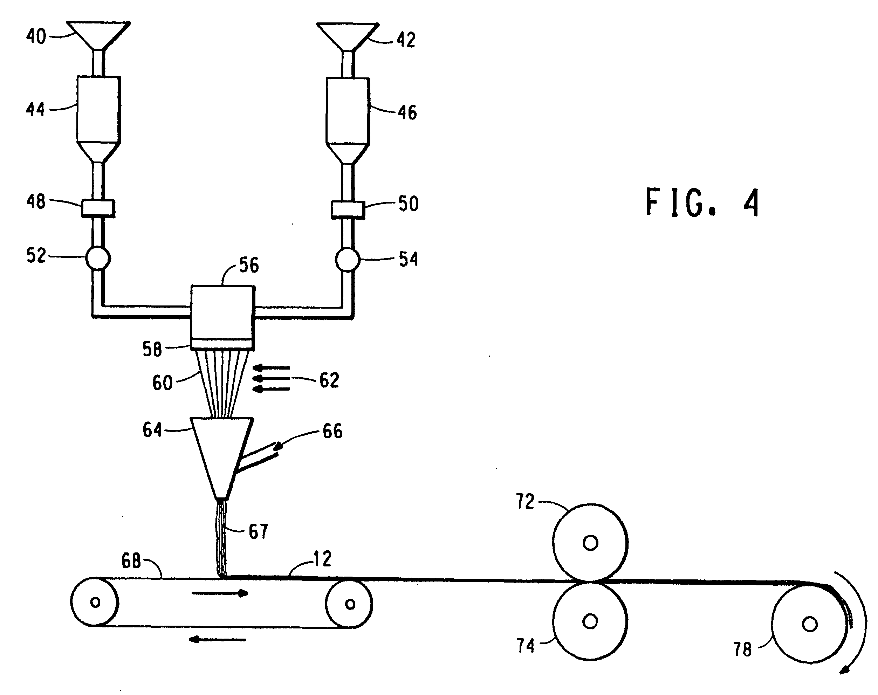

An apparatus for producing high strength bicomponent meltspun fibers at high speeds is schematically illustrated in Figure 4. In this apparatus, two thermoplastic polymers are fed into the hoppers 40 and 42, respectively. The polymer in hopper 40 is fed into the extruder 44 and the polymer in the hopper 42 is fed into the extruder 46. The extruders 44 and 46 each melt and pressurize the polymer and push it through filters 48 and 50 and metering pumps 52 and 54, respectively. The polymer from hopper 40 is combined with polymer from hopper 42 in the spin block 56 by known methods to produce the desired multiple component filament cross sections mentioned above, as for example by using a multi-component spin block like that disclosed in U.S. Patent No. 5,162,074. Where the filaments have a sheath-core cross section, a lower melting polymer is typically used for the sheath layer so as to enhance thermal bonding. If desired, single component fibers can be spun from the multicomponent apparatus shown in Figure 4 by simply putting the same polymer in both of the hoppers 40 and 42.

The melted polymers exit the spm block 56 through a plurality of capillary openings on the face of the spinneret 58. The capillary operungs may be arranged on the spinneret face in a conventional pattern (rectangular, staggered, etc.) with the spacing of the openings set to optimize productivity and fiber quenching. The density of the openings is typically in the range of 500 to 8000 holes/meter width of the pack. Typical polymer throughputs per opening are in the range of 0.3 to 5.0 g/mm.

The filaments 60 extruded from the spin block 56 are initially cooled with quenching air 62 and then drawn by a pneumatic draw jet 64 before being laid down. The quenching air is provided by one or more conventional quench boxes that direct air against the filaments at a rate of about 0.3 to 2.5 m/sec and at a temperature in the range of 5° to 25° C. Typically, two quench boxes facing each other from opposite sides of the line of filaments are used in what is known as a co-current air configuration. The distance between the capillary openings and the draw jet may be anywhere from 30 to 130 cm, depending on the fiber properties desired. The quenched filaments enter the pneumatic draw jet 64 where the filaments are drawn by air 66 to fiber speeds in the range of from 2000 to 12,000 m/min. This pulling of the filaments draws and elongates the filaments as the filaments pass through the quench zone. The filaments 67 exiting the draw jet 64 are thinner and stronger than the filaments that were extruded from the spin block. The substantially continuous fiber filaments 67 are strong fibers having a tensile strength of at least 1 gpd, and preferably having an effective diameter of from 5 to 30 microns. The filaments 67 are deposited onto a laydown belt or forming screen 68 as substantially continuous fiber filaments. The distance between the exit of the draw jet 64 and the laydown belt is vaned depending on the properties desired in the nonwoven web, and generally ranges between 13 and 76 cm. A vacuum suction is usually applied through the laydown belt to help pin the fiber web. Where desired, the resulting web 12 can be passed between thermal bonding rolls 72 and 74 before being collected on the roll 78.

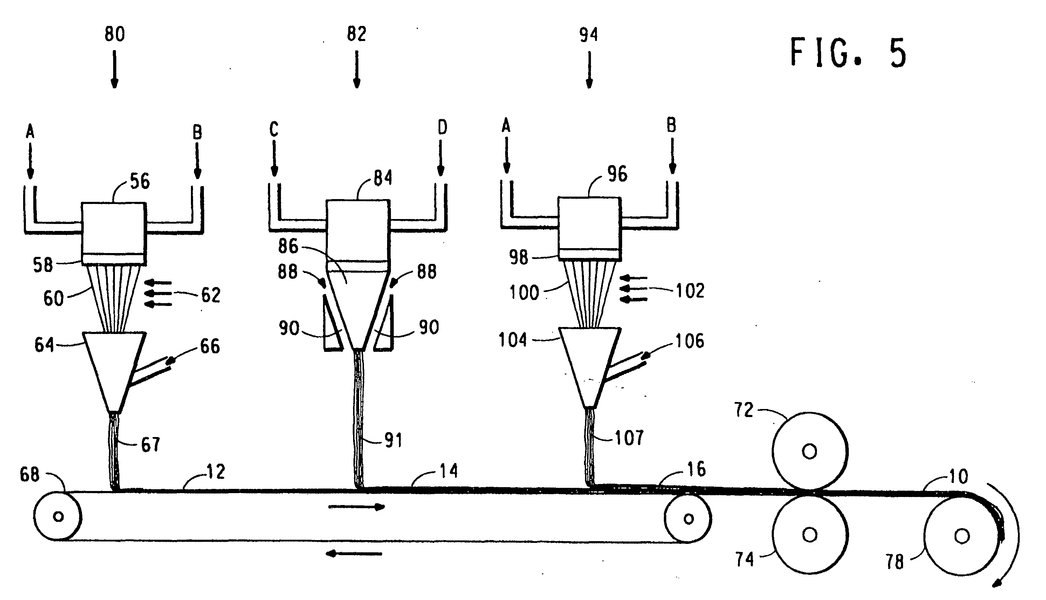

The composite nonwoven fabric of the invention can be produced in-line using the apparatus that is shown schematically in Figure 5. Alternatively, the layers of the composite sheet can be produced independently and later combined and bonded to form the composite sheet. The apparatus shown in Figure 5 includes spunbonded web production sections 80 and 94 that are preferably like the high speed melt spinning apparatus described with regard to Figure 4. The apparatus of Figure 5 further includes a meltblown web production section 82 incorporating the meltblowing apparatus described with regard to Figures 3 or 7 above. For purposes of illustration, the two spunbond web production sections 80 and 94 are shown making bicomponent fibers. It is contemplated that the spunbond web production sections 80 and 94 could be replaced by units designed to produce spunbond webs having just one polymer component or having three or more polymer components. It is also contemplated that more than one spunbond web production section could be used in series to produce a web made of a blend of different single or multiple component fibers. It is further contemplated that the polymer(s) used in section 94 could be different than the polymer(s) used in section 80. Where it is desired to produced a composite sheet having just one spunbond layer and one fine fiber layer (as shown in Figure 2), the second spunbond web production section 94 can be turned off or eliminated.

According to the preferred embodiment of the invention, in the spunbond web production sections 80 and 94 of the apparatus shown in Figure 5, two thermoplastic polymer components A and B are melted, filtered and metered (not shown) to the spin blocks 56 and 96 as described above with regard to Figure 4. The melted polymer filaments 60 and 100 are extruded from the spin blocks through spinneret sets 58 and 98, respectively, as described above with regard to Figure 4. The filaments may be extruded as bicomponent filaments having a desired cross section, such as a sheath-core filament cross section. Preferably, a lower melting temperature polymer is used for the sheath section while a higher melting temperature polymer is used for the core section. The resulting filaments 60 and 100 are cooled with quenching air 62 and 102 as described above. The filaments next enter pneumatic draw jets 64 and 104 and are drawn by drawing air 66 and 106 as described above with regard to Figure 4. The fibers 67 from the spunbond web production section 80 are deposited onto forming screen 68 so as to form a spunbond layer 12 on the belt.

According to the preferred embodiment of the invention, two thermoplastic polymers C and D are combined to make a meltblown bicomponent web in the meltblown web production section 82. Polymers C and D are melted, filtered, and then metered (not shown) into the spin block 84. The melted polymers are combined in the meltblowing spin block 84 and exit the spin block through a line of capillary openings 86 as described above. Preferably, the spin block 86 generates the desired side-by-side fiber filament cross section. Alternative spin block arrangements can be used to produce alternative fiber cross sections, such as a sheath-core cross section. A jet of hot air 88 supplied from the channels 90 impacts on the opposite side of the exiting filaments 91 and attenuates each filament 91 immediately after each filament exits its capillary opening. The meltblown filaments 91 are generally fractured dunng the attenuation process. The meltblown filament fibers 91 deposit onto spunbond layer 12 to create the multiple component meltblown web layer 14.

Where a second spunbond web production section 94 is used. substantially continuous spunbond fibers 107 from the spunbond web production section 80 may be deposited onto the meltblown layer 14 so as to form a second spunbond layer 16 on web. The layers 12 and 16 do not necessarily have to have the same thickness or basis weight.

The spunbond-meltblown-spunbond web structure is passed between thermal bonding rolls 72 and 74 in order to produce the composite nonwoven web 10 which is collected on a roll 78. Preferably, the bonding rolls 72 and 74 are heated rolls maintained at a temperature within plus or minus 20° C of the lowest melting temperature polymer in the composite. For the polyethylene-containing composite sheet of the invention, a bonding temperature in the range of 115-120 °C and a bonding pressure in the range of 350-700 N/cm has been applied to obtain good thermal bonding. Alternative methods for bonding the layers of the composite sheet include calender bonding, through-air bonding, steam bonding, and adhesive bonding.

Optionally, a fluorochemical coating can be applied to the composite nonwoven web 10 to reduce the surface energy of the fiber surface and thus increase the fabric's resistance to liquid penetration. For example, the fabric may be treated with a topical finish treatment to improve the liquid barrier and in particular, to improve barrier to low surface tension liquids. Many topical finish treatment methods are well known in the art and include spray application, roll coating, foam application, dip-squeeze application, etc. Typical finish ingredients include ZONYL® fluorochemical (available from DuPont, Wilmington, DE) or REPEARL® fluorochemical (available from Mitsubishi Int. Corp, New York, NY). A topical finishing process can be carried out either in-line with the fabnc production or in a separate process step. Alternatively, such fluorochemicals could also be spun into the fiber as an additive to the melt.

The composite nonwoven sheet 10 of the invention preferably has a basis weight in the range of 10 to 120 g/m², and more preferably within the range of 30 to 90 g/m², and most preferably within the range of 50 to 70 g/m². The grab tensile strength of the composite nonwoven sheet can range widely depending on the thermal bonding conditions employed. Typical grab tensile sheet strengths (in both the machine and cross directions) are from 35 to 400 N, and more preferably from 40 to 300 N, and most preferably from 50 to 200 N. The inner meltblown fiber layer of the composite sheet typically has a basis weight of between 2 and 40 g/m², and more preferably between 5 and 30 g/m², and most preferably between 12 and 25 g/m². The outer layer of the composite contributes strength, and is some instances barrier, to the composite nonwoven fabric. Each of the outer layers typically have a basis weight between 3 and 50 g/m², and more preferably between 8 and 40 g/m², and most preferably between 12 and 35 g/m². Preferably, the layers of the composite sheet are secured together by thermal bonding, as for example via the melting of a low melting temperature component polymer in the fine fiber layer 14 and/or the larger fiber layers 12 and 16. According to the preferred embodiment of the invention, the composite sheet exhibits a hydrostatic head of at least 10 cm, and more preferably of at least 20 cm, and most preferably of at least 35 cm. It is further preferred that the composite sheet exhibit a water impact of less than 5 g, and more preferably less than 2 g, and most preferably less than 0.5 g. Finally, it is preferred that the composite sheet have a Frazier Air Permeability greater than 1 m³/min/m², and more preferably greater than 5 m³/min/m².

This invention will now be illustrated by the following non-limiting examples which are intended to illustrate the invention and not to limit the invention in any manner.

### EXAMPLES

Composite sheets like that shown in Figure I were prepared. Each composite sheet was comprised of a layer of meltblown fibers sandwiched between spunbond outer layers. The meltblown inner layer was prepared according to the processes described above with reference to Figure 4. The meltblowing process conditions for the meltblown layer of each composite sheet are reported in Table 1. The spunbond layers were each produced individually using a high speed melt spinning process like that described above with regard to the spunbond web production section 80 of the process shown in Figure 5. However, instead of preparing all of the layers in one continuous process as described with reference to Figure 5, the spunbond layers were each spun, laid down, and rolled up separately. The two spunbond layers and the meltblown layer were subsequently unrolled and combined to produce spunbond-meltblown-spunbond web structures that were thermally bonded to produce composite nonwoven sheets. In all of the examples, bonding temperatures in the range of 115° to 120° C and bonding pressures in the range of 350 to 700 N/cm were applied. It has been found that variation of bonding conditions within these bonding temperature and pressure ranges does not have a significant effect on the bamer or tensile properties of the bonded composite sheet. Accordingly, bonding conditions for the examples below are reported in Table 2 but are not specifically discussed for each example. The properties measured on the composite sheet of each example are reported in Table 2.

### EXAMPLES 1, 1a & 1b

A meltblown bicomponent web was made with a polyethylene component and a poly(ethylene terephthalate) component. The polyethylene component was made from linear low density polyethylene with a melt index of 150 g/10 minutes (measured according to ASTM D-1238) available from Dow as ASPUN 6831A. The polyester component was made from poly(ethylene terephthalate) with an intrinsic viscosity of 0.53 (as measured in U.S. Patent 4,743,504) available from DuPont as Crystar® polyester (Merge 3949). The polyester polymer was crystallized and dried prior to extrusion. The polyethylene polymer was heated to 450° F (232 °C) and the polyester polymer was heated to 572° F (300° C) in separate extruders. The two polymers were separately extruded, filtered and metered to a bicomponent spin block arranged to provide a side-by-side filament cross section. The die of the spin block was heated to 572 °F (300°C). The die had 601 capillary openings arranged in a 24 inch (61 cm) line. The polymers were spun through the each capillary at a polymer throughput rate of 0.40 g/hole/min. Attenuating air was heated to a temperature of 600° F (316° C) and supplied at a rate of 400 standard cubic feet per minute (11.33 m³/min) through two 0.8 mm wide air channels. The two air channels ran the length of the 24 inch line of capillary openings, with one channel on each side of the line of capillaries set back 1mm from the capillary openings. The polyethylene was supplied to the spin block at a rate of 2.9 kg/hr and the polyester was supplied to the spin block at a rate of 11.7 kg/hr. A bicomponent meltblown web was produced that was 20 weight percent polyethylene and 80 weight percent polyester. The filaments were collected on a moving forming screen to produce a meltblown web. The meltblown web was collected on a roll. The meltblown web had a basis weight of 11.7 g/m².

The spunbond outer layers were bicomponent fibers with a sheath-core cross section. The spunbond fibers were made using an apparatus like that described above with regard to Figure 4. Spunbond webs with two basis weights (15 g/m² and 30 g/m²) were produced for use in the outer layers of the composite sheet. The spunbond bicomponent fibers were made from linear low density polyethylene with a melt index of 27 g/10 minutes (measured according to ASTM D-1238) available from Dow as ASPUN 6811A, and poly(ethylene terephthalate) polyester with an intrinsic viscosity of 0.53 (as measured in U.S. Patent 4,743,504) available from DuPont as Crystar® polyester (Merge 3949). The polyester resin was crystallized at a temperature of 180° C and dried at a temperature of 120° C to a moisture content of less than 50 ppm before use.

The polyester was heated to 290° C and the polyethylene was heated to 280° C in separate extruders. The polymers were extruded, filtered and metered to a bicomponent spin block maintained at 295° C and designed to provide a sheath-core filament cross section. The polymers were spun through the spinneret to produce bicomponent filaments with a polyethylene sheath and a poly(ethylene terephthalate) core. The total polymer throughput per spin block capillary was 0.4 g/min for the 15 g/m² basis weight web and 0.5g/min for the 30 g/m² web. The polymers were metered to provide filament fibers that were 30% polyethylene (sheath) and 70% polyester (core), based on fiber weight. The filaments were cooled in a 15 inch (38.1 cm) long quenching zone with quenching air provided from two opposing quench boxes a temperature of 12° C and velocity of 1 m/sec. The filaments passed into a pneumatic draw jet spaced 20 inches (50.8 cm) below the capillary openings of the spin block where the filaments were drawn at a rate of approximately 9000 m/min. The resulting smaller, stronger substantially continuous filaments were deposited onto a laydown belt with vacuum suction. The fibers in the two webs (15 g/m² and 30 g/m² basis weights) had an effective diameter in the range of 6 to 8 microns. The resulting webs were passed between two thermal bonding rolls to lightly tack the web together for transport using a point bonding pattern at a temperature of 100° C and a nip pressure of 100 N/cm. The line speed during bonding was 150 m/min for the 15 g/m² basis weight web and 100 m/min for the 30 g/m² basis weight web. The lightly bonded spunbond webs were each collected on a roll.

The composite nonwoven sheet was prepared by unrolling the 15 g/m² basis weight spunbond web onto a moving belt. The meltblown bicomponent web was unrolled and laid on top of the moving spunbond web. A second roll of the 15 g/m² basis weight spunbond web was unrolled and laid on top of the spunbond-meltblown web to produce a spunbond-meltblown-spunbond composite nonwoven web. The composite web was thermally bonded between an engraved oil-heated metal calender roll and a smooth oil heated metal calender roll. Both rolls had a diameter of 466 mm. The engraved roll had a chrome coated non-hardened steel surface with a diamond pattern having a point size of 0.466mm², a point depth of 0.86 mm, a point spacing of 1.2 mm, and a bond area of 14.6%. The smooth roll had a hardened steel surface. The composite web was bonded at a temperature of 120 °C, a nip pressure of 350 N/cm, and a line speed of 50 m/min. The bonded composite sheet was collected on a roll. This composite nonwoven sheet, made from two layers of 15 g/m² basis weight spunbonded webs and the bicomponent meltblown web. is the composite sheet of Example 1a. The final basis weight of this composite nonwoven sheet was 42.1 g/m².

In Example 1b, another composite nonwoven sheet was made using identical bonding conditions, but using two layers of 30 g/m² basis weight spunbonded webs in place of the 15 g/m² webs of Example 1a. The final basis weight of this composite nonwoven sheet of Example 1b was 65.7 g/m².

### EXAMPLE 2

A composite sheet was formed according to the procedure of Example 1 except that the speed of the forming screen onto which the meltblown web was deposited was adjusted so as to increase the basis weight of the meltblown layer to 16.8 g/m². The outer layers of the composite sheet were both made of the 30 g/m² basis weight spunbond web described in Example 1. The physical properties of the sheet are reported in Table 2.

### EXAMPLE 3

A composite sheet was formed according to the procedure of Example 2 except that speed of the forming screen onto which the meltblown web was deposited was adjusted so as to increase the basis weight of the meltblown layer to 24.1 g/m². The physical properties of the sheet are reported in Table 2.

### EXAMPLES 4, 4a & 4b

Composite sheets were formed according to the procedure of Examples 1, 1a and 1b except the meltblowing process was altered as follows: the attenuating air flow was increased to 500 scfm (14.16 m³/min); the polymer throughput was increased to 0.75 g/hole/min; the polyethylene feed rate was increased to 11.4 kg/hr while the poly(ethylene terephthalate) feed rate was increased to 15.6 kg/hr to obtain a meltblown web that was 42% by weight polyethylene and 58% by weight polyester; and speed of the forming screen was adjusted to obtain a meltblown web with a basis weight of 11.0 g/m². The physical properties of the sheets are reported in Table 2.

### EXAMPLE 5

A composite sheet was formed according to the procedure of Example 2 except the meltblowing process was altered as follows: the attenuating air flow was changed to 350 scfm (9.91 m²/min); the polymer throughput was changed to 0.37 g/hole/min; the polyethylene feed rate was changed to 5.7 kg/hr while the poly(ethylene terephthalate) feed rate was changed to 7.8 kg/hr to obtain a meitblown web that was 42% by weight polyethylene and 58% by weight polyester; and speed of the forming screen was adjusted to obtain a meltblown web with a basis weight of 16.3 g/m². The physical properties of the sheet are reported in Table 2.

The composite nonwoven sheet was further treated with a standard fluorochemical finish to reduce the surface energy of the fiber surface, and thus increase the fabric's resistance to liquid penetration. The sheet was dipped into an aqueous bath of 4 weight % Repearl® F-35 fluorochemical (obtained from Mitsubishi), 0.25 weight % Zelec® TY antistat (obtained from DuPont), and 20 weight % isopropanol, a wetting agent commercially available from many sources. The webs were then squeezed to remove excess liquid and dried and cured in an oven for 2 minutes at 105 °C. The physical properties of fluorochemically treated nonwoven sheet are reported in Table 3.

### EXAMPLE 6

A composite sheet was formed according to the procedure of Example 2 except the meltblowing process was altered as follows: the attenuating air flow rate was changed to 500 scfm (14.16 m³/min) and air flow temperature was changed to 560° F (293° C); the polymer throughput was changed to 0.75 g/hole/min; the polyethylene feed rate was changed to 11.4 kg/hr while the poly(ethylene terephthalate) feed rate was changed to 15.6 kg/hr to obtain a meltblown web that was 42% by weight polyethylene and 58% by weight polyester; and speed of the forming screen was adjusted to obtain a meltblown web with a basis weight of 21.7 g/m². The physical properties of the sheet are reported in Table 2.

The composite nonwoven web was further treated with a the fluorochemical finish as described in Example 5. The physical properties of fluorochemically treated nonwoven sheet are reported in Table 3.

### EXAMPLES 7, 7a & 7b

Composite sheets were formed according to the procedure of Examples 1, 1a and 1b except the meltblowing process was altered as follows: the attenuating air flow was changed to 300 scfm (8.50 m³/min); the polyethylene feed rate was changed to 11.7 kg/hr while the poly(ethylene terephthalate) feed rate was changed to 11.0 kg/hr to obtain a molten web that was 80% by weight polyethylene and 20% by weight polyester; and speed of the forming screen was adjusted to obtain a meltblown web with a basis weight of 11.4 g/m². The physical properties of the sheets are reported in Table 2.

### EXAMPLE 8

A composite sheet was formed according to the procedure of Example 2 except the meltblowing process was altered as follows: the attenuating air flow rate was changed to 300 scfm (8.50 m³/min); the polyethylene feed rate was changed to 11.7 kg/hr while the poly(ethylene terephthalate) feed rate was changed to 2.8 kg/hr to obtain a meltblown web that was 80% by weight polyethylene and 20% by weight polyester; and speed of the forming screen was adjusted to obtain a meltblown web with a basis weight of 15.0 g/m². The physical properties of the sheet are reported in Table 2.

The composite nonwoven web was further treated with the fluorochemical finish as described in Example 5. The physical properties of fluorochemically treated nonwoven sheet are reported in Table 3.

### EXAMPLE 9

A composite sheet was formed according to the procedure of Example 2 except the meltblowing process was altered as follows: the attenuating air flow rate was changed to 300 scfm (8.50 m³/min); the polyethylene feed rate was changed to 11.7 kg/hr while the poly(ethylene terephthalate) feed rate was changed to 2.8 kg/hr to obtain a meltblown web that was 80% by weight polyethylene and 20% by weight polyester; and speed of the forming screen was adjusted to obtain a meltblown web with a basis weight of 22.2 g/m². The physical properties of the sheet are reported in Table 2.

The composite nonwoven web was further treated with the fluorochemical finish as described in Example 5. The physical properties of fluorochemically treated nonwoven sheet are reported in Table 3.

### EXAMPLE 10

A composite sheet was formed according to the procedure of Examples 1, 1a and I b except the polyester component was made from poly(ethylene terephthalate) with an intrinsic viscosity of 0.53 (as measured in U.S. Patent 4,743,504) available from DuPont as Crystar® polyester (Merge 4449). Crystar® Merge 4449 is a crystallized version of Crystar® Merge 3949. Also, the meltblowing process was altered as follows: the die temperature was changed to 315° C; the attenuating air flow temperature was changed to 322° C; the attenuating air flow rate was changed to 420 scfm (714 m³/min); the polymer throughput was changed to 0.80 g/hole/min; the polyethylene feed rate was changed to 23.1 kg/hr while the poly(ethylene terephthalate) feed rate was changed to 5.8 kg/hr to obtain a meltblown web that was 80% by weight polyethylene and 20% by weight polyester; and speed of the forming screen was adjusted to obtain a meltblown web with a basis weight of 17.5 g/m². The physical properties of the sheet are reported in Table 2.

### EXAMPLE 11

A composite sheet was formed according to the procedure of Example 10 except the polyethylene component was made up of a blend of 90% by weight Dow ASPUN 6831A and 10% by weight Hoechst Celanese 1300A poly(butyl terephthalate). The poly(butyl terephthalate) acts as a spinning aid to the polyethylene. Also, the meltblowing process was altered as follows: the polyethylene/poly(butyl terephthalate) blend was heated to 260° C and the attenuating air flow rate was changed to 425 scfm (722 m³/min). The physical properties of the sheet are reported in Table 2.

Comparing Example 11 with Example 10 shows that the hydrostatic head is higher for Example 11 with the poly(butyl terephthalate) in the polyethylene component than Example 10 which has no poly(butyl terephthalate) in the polyethylene component.

### COMPARATIVE EXAMPLE A

The procedure of Example 1 was followed except in that the meltblown web. the poly(ethylene terephthalate) component was replaced with the same polyethylene used for the other component. This modification produced a single component polyethylene meltblown fiber web. Other changes in meltblown process conditions are as shown in Table 1. The hydrostatic head exhibited by the composite sheet of Comparative Example A was significantly lower than that of the composite sheets with comparable basis weights that were made according to the invention.

### COMPARATIVE EXAMPLE B

A composite sheet was formed according to the process of Comparative Example A except that the meltblowing process was altered as follows: the attenuating air flow rate was changed to 250 scfm (7.08 m³/min); and speed of the forming screen was adjusted to obtain a meltblown web with a basis weight of 19.7 g/m². The hydrostatic head exhibited by the composite sheet of Comparative Example B was significantly lower than that of the composite sheets with comparable basis weights that were made according to the invention.

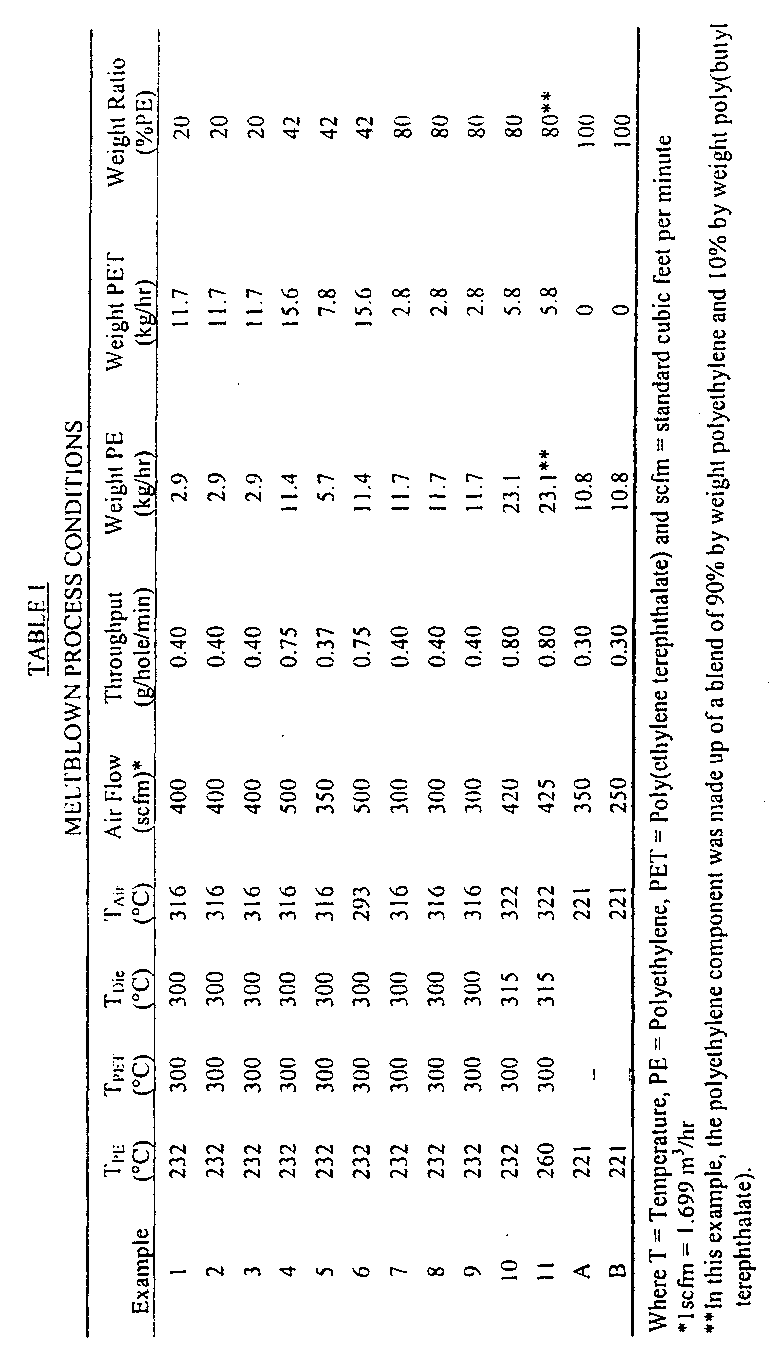

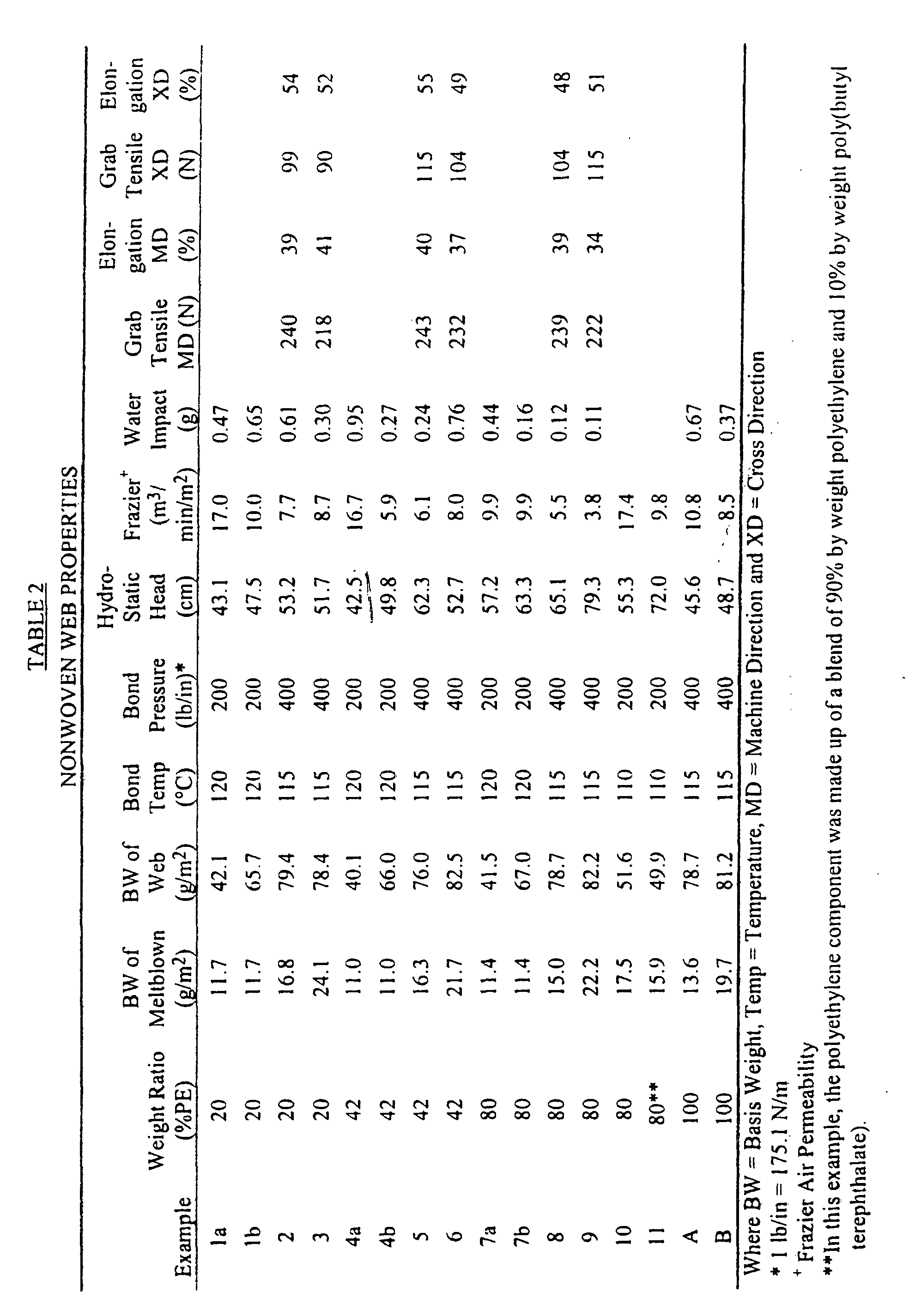

**TABLE 3**

| FLUOROCHEMICALLY TREATED COMPOSITE NONWOVEN WEB | | |
|---|---|---|
| BARRIER PROPERTIES | | |
| Examples | Hydro-Static Head (cm) | Water Impact (g) |
| 5 | 48.5 | 0.01 |
| 6 | 40.0 | 0.01 |
| 8 | 53.0 | 0.01 |
| 9 | 62.0 | 0.01 |

### EXAMPLES 12a - 12k

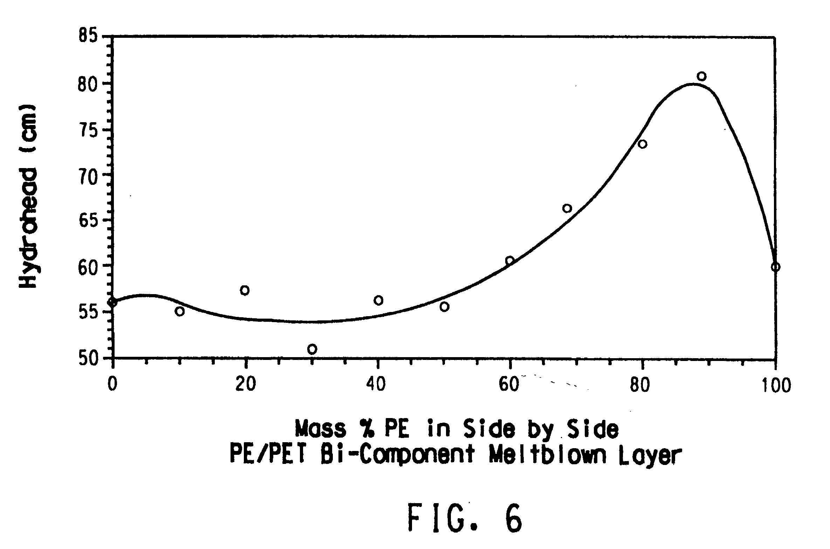

Composite sheets were formed according to the procedure of Example 1 in order to produce a range of nonwoven sheets with different amounts of polyethylene and polyester polymer components in the bicomponent meltblown web layer of the sheets, except that the meltblowing process was altered as follows: the attenuating air flow was increased to 300 scfin (8.50 m³/min); the polyethylene feed rate and the poly(ethylene terephthalate) feed rate were adjusted as reported in Table 4 to achieve a variety of polymer component ratios in the fibers of the meltblown layer; and the speed of the forming screen was adjusted to obtain a meltblown web with a basis weight of 22 g/m². The outer spunbond layers were each made of the 15 g/m² basis weight spunbond web described in Example 1. For each of the spunbond-meltblown-spunbond composite nonwoven sheets of Examples 12a - 12k, bonding was performed at a bonding temperature of 110 °C and a bonding line speed of 10 m/min. The physical properties of the sheets are reported in Table 4. Figure 6 is a graph ratio of the weight percent of the polyethylene component to the polyester component in the meltblown fibers vs. the hydrostatic head measured on each composite sheet sample.

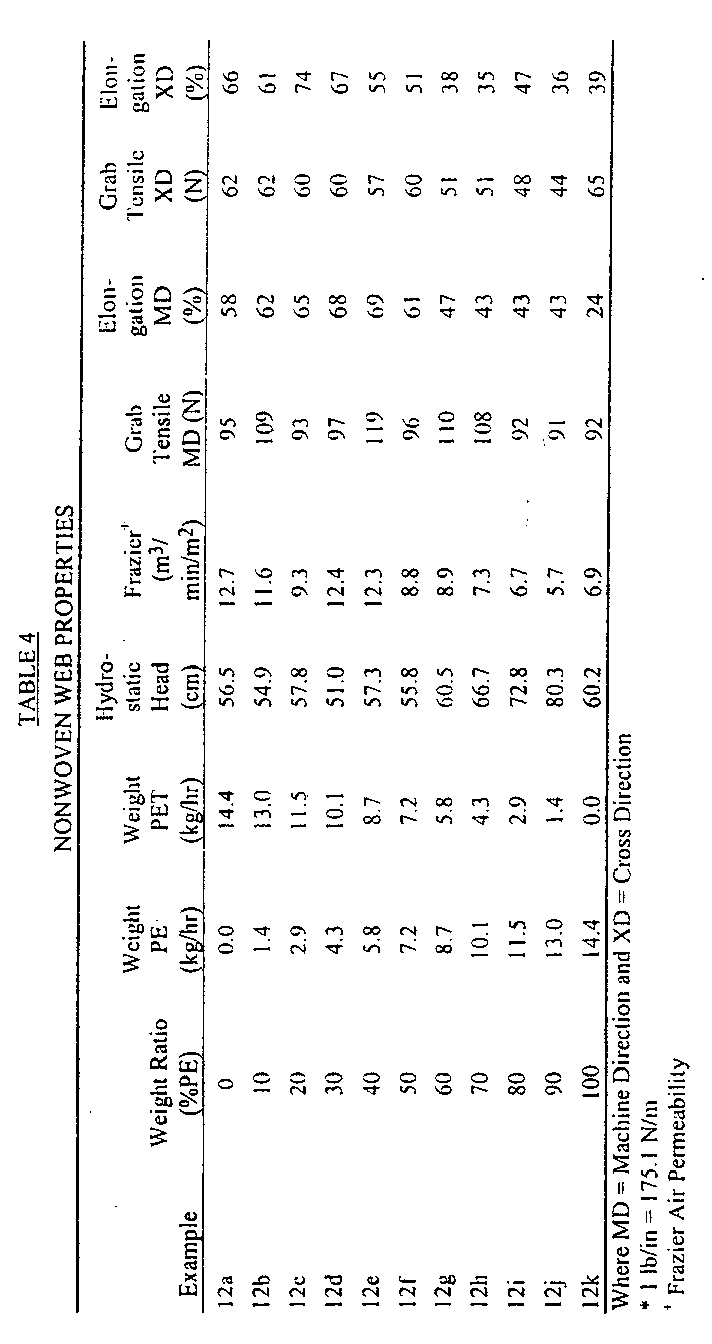

## Claims

1. A composite sheet comprising:
a first fibrous layer, a second fibrous layer and a third fibrous layer, said first fibrous layer being thermally bonded between said second and third fibrous layers;
said first fibrous layer being a multiple component meltblown web comprised of at least 95% by weight of meltblown fibers having an average effective diameter of less than 10 microns, said multiple component meltblown web comprised of 10% to 98% by weight of a first polymer component and 80% to 2% by weight of a second polymer component distinct from said first polymer component, said first polymer component being polyethylene and said second polymer component being a polyester polymer;
said second and third fibrous layer each comprised of at least 95% by weight of second layer meltspun fibers having an average effective diameter that is greater than the average effective diameter of said first layer fibers;
said composite sheet having a basis weight of less than 120 g/m², a machine direction and cross direction grab tensile strength of at least 35 N, and a hydrostatic head of at least 42.5 cm.

2. The composite sheet of claim 1 wherein said multiple component meltblown web of said first fibrous layer is comprised of between 20% and 98% by weight of said first polymer component and between 80% and 2% by weight of said second polymer component.

3. The composite sheet of claim I wherein said multiple component meltblown web of said first fibrous layer is comprised of between 55% and 98% by weight of said first polymer component and between 45% and 2% by weight of said second polymer component.

4. The composite sheet of claim 1 wherein said multiple component meltblown web of said first fibrous layer is comprised of between 65% and 97% by weight of said first polymer component and between 35% and 3% by weight of said second polymer component.

5. The composite sheet of claim 1 wherein said multiple component meltblown web of said first fibrous layer is comprised of between 80% and 95% by weight of said first polymer component and between 20% and 5% by weight of said second polymer component.

6. The composite sheet of claim 1 wherein said polyester polymer is selected from the group of poly(ethylene terephthalate), poly(trimethylene terephthalate), poly(butylene terephthalate), and copolymers and terpolymers thereof.

7. The composite sheet of claim 6 wherein said polyester polymer is poly(ethylene terephthalate).

8. The composite sheet of claim 1 wherein at least 10% of the fibers in said multiple component meltblown web are bicomponent fibers having a length, and wherein the first and second polymer components are arranged in such bicomponent fibers in a manner such that said first and second polymer components each extend substantially the complete length of said bicomponent fibers.

9. The composite sheet of claim 8 wherein said first and second polymer components of said bicomponent fibers are arranged in a side-by-side arrangement wherein said first and second polymer components each extend substantially the complete length of said first layer fibers.

10. The composite sheet of claim 1 wherein said second and third layer fibers are substantially continuous meltspun fibers having an average effective diameter of at least 5 microns.

11. The composite sheet of claim 10 wherein said second and third layer fibers are multiple component meltspun fibers including a polyethylene polymer and another distinct second polymer component, said polyethylene polymer component comprising at least 5% by weight of said second layer fibers.

12. The composite sheet of claim 11 wherein the second polymer component of said second and third layer fibers is selected from the group of polyester, polyamide, polyurethane, polypropylene, and polystyrene polymers.

13. The composite sheet of claim 12 wherein the fibers of said first, second and third fibrous layers are gamma radiation sterilizable.

14. The composite sheet of claim 12 wherein said meltspun fibers having an average effective diameter in the range of 6 to 10 microns and a machine direction and cross direction grab tensile strength of at least 35 Newtons.

15. The composite sheet according to claim 14 wherein second and third layers have hydrostatic heads of at least 3 cm.

16. The composite sheet of claim 14 wherein said meltspun fibers are sheath core bicomponent fibers having a polyethylene sheath and a polyester core.

17. The composite sheet of claim 1 wherein said composite sheet has a basis weight in the range of 30 to 90 g/m², a machine direction and cross direction grab tensile strength of at least 40 N.

18. The composite sheet of claim 17 wherein said composite sheet has a basis weight of less than 70 g/m².

19. The composite sheet of claim 17 wherein said composite sheet has a water impact of less than 5 g.

20. The composite sheet of claim 17 wherein said composite sheet has a water impact of less than 2 g, and a Frazier Air Permeability of at least I m³/min/m².

21. A garment comprised of the composite sheet of claim 1.

## Patentansprüche

1. Flächiger Verbundstoff, aufweisend:
eine erste Faserlage, eine zweite Faserlage und eine dritte Faserlage, wobei die erste Faserlage zwischen den zweiten und dritten Faserlagen thermisch gebondet ist;
die erste Faserlage ist eine mehrkomponentige, schmelzgeblasene Faserbahn, die mindestens 95 Gew.% der schmelzgeblasenen Fasern mit einem mittleren wirksamen Durchmesser von weniger als 10 µm aufweist, wobei die mehrkomponentige, schmelzgeblasene Faserbahn 10% bis 98 Gew.% einer ersten Polymerkomponente aufweist und 80% bis 2 Gew.% einer zweiten Polymerkomponente, die von der ersten Polymerkomponente verschieden ist, wobei die erste Polymerkomponente Polyethylen ist und die zweite Polymerkomponente Polyester-Polymer ist;
wobei die zweite und dritte Faserlage jeweils mindestes 95 Gew.% schmelzgesponnene Fasern der zweiten Lage mit einem mittleren wirksamen Durchmesser aufweist, der größer ist als der mittlere wirksame Durchmesser der Fasern der ersten Lage;
wobei der flächige Verbundstoff ein flächenbezogenes Gewicht von weniger als 120 g/m² hat, eine Einspannzugfestigkeit in Faserlaufrichtung und Querrichtung von mindestens 35 N und einen hydrostatischen Druckwert von mindestens 42,5 cm.

2. Flächiger Verbundstoff nach Anspruch 1, bei welchem die mehrkomponentige, schmelzgeblasene Faserbahn der ersten Faserlage zwischen 20% und 98 Gew.% der ersten Polymerkomponente aufweist und zwischen 80% und 2 Gew.% der zweiten Polymerkomponente aufweist.

3. Flächiger Verbundstoff nach Anspruch 1, bei welchem die mehrkomponentige, schmelzgeblasene Faserbahn der ersten Faserlage zwischen 55% und 98 Gew.% der ersten Polymerkomponente aufweist und zwischen 45% und 2 Gew.% der zweiten Polymerkomponente aufweist.

4. Flächiger Verbundstoff nach Anspruch 1, bei welchem die mehrkomponentige, schmelzgeblasene Faserbahn der ersten Faserlage zwischen 65% und 97 Gew.% der ersten Polymerkomponente aufweist und zwischen 35% und 3 Gew.% der zweiten Polymerkomponente aufweist.

5. Flächiger Verbundstoff nach Anspruch 1, bei welchem die mehrkomponentige, schmelzgeblasene Faserbahn der ersten Faserlage zwischen 80% und 95 Gew.% der ersten Polymerkomponente aufweist und zwischen 20% und 5 Gew.% der zweiten Polymerkomponente aufweist.

6. Flächiger Verbundstoff nach Anspruch 1, bei welchem das Polyester-Polymer ausgewählt ist aus der Gruppe, bestehend aus Poly(ethylenterephthalat), Poly(trimethylenterephthalat), Poly(butylenterephthalat) und Copolymeren und Terpolymeren davon.

7. Flächiger Verbundstoff nach Anspruch 6, bei welchem das Polyester-Polymer Poly(ethylenterephthalat) ist.

8. Flächiger Verbundstoff nach Anspruch 1, bei welchem mindestens 10% der Fasern in der mehrkomponentigen, schmelzgeblasenen Faserbahn Bikomponentenfasern sind, die eine Länge haben und worin die ersten und zweiten Polymerkomponenten in den Bikomponentenfasern in einer solchen Form angeordnet sind, dass die ersten und zweiten Polymerkomponenten jeweils wesentlich über die volle Länge der Bikomponentenfasern hinausragen.

9. Flächiger Verbundstoff nach Anspruch 8, bei welchem die ersten und zweiten Polymerkomponenten der Bikomponentenfasern in einer Seite-an-Seite-Anordnung angeordnet sind, worin die ersten und zweiten Polymerkomponenten jeweils wesentlich über die volle Länge der Fasern der ersten Lage hinausragen.

10. Flächiger Verbundstoff nach Anspruch 1, bei welchem die Fasern der zweiten und dritten Lage weitgehend schmelzgesponnene Endlosfasern sind, die einen mittleren wirksamen Durchmesser von mindestens 5 µm haben.

11. Flächiger Verbundstoff nach Anspruch 10, bei welchem die Fasern der zweiten und dritten Lage mehrkomponentige, schmelzgesponnene Fasern sind, in die eine Polyethylen-Polymerkomponente und eine andere, verschiedene zweite Polymerkomponente einbezogen ist, wobei die Polyethylen-Polymerkomponente mindestens 5 Gew.% der Fasern der zweiten Lage ausmacht.

12. Flächiger Verbundstoff nach Anspruch 11, bei welchem die zweite Polymerkomponente der Fasern der zweiten und dritten Lage ausgewählt ist aus der Gruppe, bestehend aus Polyester-, Polyamid-, Polyurethan-, Polypropylen- und Polystyrol-Polymere.

13. Flächiger Verbundstoff nach Anspruch 12, bei welchem die Fasern der ersten, zweiten und dritten Faserlagen durch Gammastrahlung sterilisiert werden können.

14. Flächiger Verbundstoff nach Anspruch 12, bei welchem die schmelzgesponnenen Fasern einen mittleren wirksamen Durchmesser im Bereich von 6 bis 10 µm haben und eine Einspannzugfestigkeit in Faserlaufrichtung und Querrichtung von mindestens 35 Newton.

15. Flächiger Verbundstoff nach Anspruch 14, bei welchem die zweiten und dritten Lagen einen hydrostatischen Druckwert von mindestens 3 cm haben.

16. Flächiger Verbundstoff nach Anspruch 14, bei welchem die schmelzgesponnenen Fasern Mantel-Kern-Bikomponentenfasern mit einem Polyethylen-Mantel und einem Polyesterkern sind.

17. Flächiger Verbundstoff nach Anspruch 1, bei welchem der flächige Verbundstoff ein flächenbezogenes Gewicht im Bereich von 30 bis 90 g/m² hat, eine Einspannzugfestigkeit in Faserlaufrichtung und Querrichtung von mindestens 40 N.

18. Flächiger Verbundstoff nach Anspruch 17, bei welchem der flächige Verbundstoff ein flächenbezogenes Gewicht von weniger als 70 g/m² hat.

19. Flächiger Verbundstoff nach Anspruch 17, bei welchem der flächige Verbundstoff einen "Wasseraufprall" von weniger als 5 g hat.

20. Flächiger Verbundstoff nach Anspruch 17, bei welchem der flächige Verbundstoff einen "Wasseraufprall" von weniger als 2 g hat und eine Luftdurchlässigkeit nach Frazier von mindestens 1 m³/min/m².

21. Bekleidung, die den flächigen Verbundstoff nach Anspruch 1 aufweist.

## Revendications

1. Feuille composite, constituée par :
une première couche fibreuse, une deuxième couche fibreuse et une troisième couche fibreuse, ladite première couche fibreuse étant liée thermiquement entre ladite deuxième et ladite troisième couche fibreuse ;
ladite première couche fibreuse étant un voile fabriqué par fusion-soufflage à composants multiples constitué par au moins 95% en poids de fibres fabriquées par fusion-soufflage présentant un diamètre effectif moyen inférieur à 10 microns, ledit voile fabriqué par fusion-soufflage à composants multiples étant constitué par 10% à 98% en poids d'un premier composant polymère et par 80% à 2% en poids d'un deuxième composant polymère différent dudit premier composant polymère, ledit premier composant polymère étant du polyéthylène et
ledit deuxième composant polymère étant un polymère polyester ;
ladite deuxième et ladite troisième couches fibreuses étant constituées chacune par au moins 95% en poids de la deuxième couche de fibres filées à l'état fondu de la deuxième couche, présentant un diamètre effectif moyen qui est supérieur au diamètre effectif moyen des fibres de ladite première couche ;
ladite feuille composite présentant un poids surfacique inférieur à 120 g/m², une résistance à la traction par la méthode d'arrachement dans le sens machine et le sens travers d'au moins 35 N et une tête hydrostatique d'au moins 42,5 cm.

2. Feuille composite selon la revendication 1, dans laquelle ledit voile fabriqué par fusion-soufflage à composants multiples de ladite première couche fibreuse est constitué par entre 20% et 98% en poids dudit premier composant polymère et entre 80% et 2% en poids dudit deuxième composant polymère.

3. Feuille composite selon la revendication 1, dans laquelle ledit voile fabriqué par fusion-soufflage à composants multiples de ladite première couche fibreuse est constitué par entre 55% et 98% en poids dudit premier composant polymère et entre 45% et 2% en poids dudit deuxième composant polymère.

4. Feuille composite selon la revendication 1, dans laquelle ledit voile fabriqué par fusion-soufflage à composants multiples de ladite première couche fibreuse est constitué par entre 65% et 97% en poids dudit premier composant polymère et entre 35% et 3% en poids dudit deuxième composant polymère.

5. Feuille composite selon la revendication 1, dans laquelle ledit voile fabriqué par fusion-soufflage à composants multiples de ladite première couche fibreuse est constitué par entre 80% et 95% en poids dudit premier composant polymère et entre 20% et 5% en poids dudit deuxième composant polymère.

6. Feuille composite selon la revendication 1, dans laquelle ledit polymère de polyester est choisi dans le groupe poly(téréphtalate d'éthylène), poly(téréphtalate de triméthylène), poly(téréphtalate de butylène) et les copolymères et les terpolymères de ceux-ci.

7. Feuille composite selon la revendication 6, dans laquelle ledit polymère de polyester est le poly(téréphtalate d'éthylène).

8. Feuille composite selon la revendication 1, dans laquelle au moins 10% des fibres dans ledit voile fabriqué par fusion-soufflage à composants multiples sont des fibres à deux composants, présentant une longueur et dans laquelle le premier et le deuxième composant polymère sont agencés dans ces fibres à deux composants de telle manière que ledit premier et ledit deuxième composant polymère s'étendent chacun substantiellement sur toute la longueur desdites fibres à deux composants.

9. Feuille composite selon la revendication 8, dans laquelle ledit premier et ledit deuxième composant polymère desdites fibres à deux composants sont agencés dans une disposition côte à côte, dans laquelle ledit premier et ledit deuxième composant polymère s'étendent chacun substantiellement sur toute la longueur desdites fibres de la première couche.

10. Feuille composite selon la revendication 1, dans laquelle lesdites fibres de la deuxième et de la troisième couche sont des fibres substantiellement continues, filées à l'état fondu présentant un diamètre effectif moyen d'au moins 5 microns.

11. Feuille composite selon la revendication 10, dans laquelle lesdites fibres de la deuxième et de la troisième couche sont des fibres filées à l'état fondu à plusieurs composants comprenant un polymère de polyéthylène et un deuxième composant polymère différent, ledit composant polymère de polyéthylène représentant au moins 5% en poids desdites fibres de la deuxième couche.

12. Feuille composite selon la revendication 11, dans laquelle le deuxième composant polymère desdites fibres de la deuxième et troisième couche est choisi dans le groupe des polymères de polyester, polyamide, polyuréthane, polypropylène et polystyrène.

13. Feuille composite selon la revendication 12, dans laquelle les fibres desdites première, deuxième et troisième couche fibreuse sont stérilisables par rayonnement gamma.

14. Feuille composite selon la revendication 12, dans laquelle lesdites fibres filées à l'état fondu présentent un diamètre effectif moyen dans la plage de 6 à 10 microns et une résistance à la traction par la méthode d'arrachement dans le sens machine et le sens travers d'au moins 35 Newtons.

15. Feuille composite selon la revendication 14, dans laquelle la deuxième et la troisième couche présentent des têtes hydrostatiques d'au moins 3 cm.

16. Feuille composite selon la revendication 14, dans laquelle lesdites fibres filées à l'état fondu sont des fibres à deux composants gaine-noyau présentant une gaine en polyéthylène et un noyau en polyester.

17. Feuille composite selon la revendication 1, dans laquelle ladite feuille composite présente un poids surfacique dans la plage de 30 à 90 g/m², une résistance à la traction par la méthode d'arrachement dans le sens machine et le sens travers d'au moins 40 N.

18. Feuille composite selon la revendication 17, dans laquelle ladite feuille composite présente un poids surfacique inférieur à 70 g/m².

19. Feuille composite selon la revendication 17, dans laquelle ladite feuille composite présente un impact de l'eau inférieur à 5 g.

20. Feuille composite selon la revendication 17, dans laquelle ladite feuille composite présente un impact de l'eau inférieur à 2 g et une perméabilité à l'air Frazier d'au moins 1 m³/min/m².

21. Vêtement constitué par la feuille de la revendication 1.
